# EUROPEAN PATENT APPLICATION

(11) **EP 2 543 311 A1**
(43) Date of publication of application: **09.01.2013**
(21) Application number: 12175209.1
(22) Date of filing: 05.07.2012
(51) Int. Cl.: A61B 5/053, A61B 5/05, A61B 5/00, A61B 8/13, G01N 21/00, G01N 22/00, G01N 27/02, G01N 29/06, G01N 33/00

(54) **System and method for excitation generation in soft-field tomography**

(30) Priority: 08.07.2011 US 201113179179
(71) Applicant: GENERAL ELECTRIC COMPANY, Schenectady, NY 12345 (US)
(72) Inventor: Ross, Alexander Seth, Niskayuna, NY New York 12309 (US); Tiwari, Arvind, Niskayuna, NY New York 12309 (US); Meethal, Manoj Kumar Koyithitta, Niskayuna, NY New York 12309 (US); Panicker, Somakumar Ramachandra, Niskayuna, NY New York 12309 (US); Mahalingam, Sakethraman, Niskayuna, NY New York 12309 (US)
(74) Representative: Illingworth-Law, William Illingworth

(57) **Abstract**

A system and method for excitation generation in soft-field tomography are provided. One method includes applying a plurality of phase modulated (or phase and amplitude modulated) excitations to a plurality of transducers of a data acquisition system positioned proximate a surface of an object and measuring a response to the applied phase modulated (or phase and amplitude modulated) excitations at the plurality of transducers. The method also includes determining a property of the object based on the measured responses.

## Description

### BACKGROUND

The subject matter disclosed herein relates generally to soft-field tomography systems and methods, and more particularly to systems and methods to generate soft-field tomography excitations.

Soft-field tomography, such as Electrical Impedance Spectroscopy (EIS) (also referred to as Electrical Impedance Tomography (EIT)), diffuse optical tomography, elastography, thermography, microwave tomography, and related modalities may be used to measure the internal properties of an object, such as the electrical properties of materials comprising internal structures of an object (e.g., a region of a human body). For example, in EIS systems, an estimate is made of the distribution of electrical conductivities of the internal structures. Such EIS systems reconstruct the conductivity and/or permittivity of the materials within the area or volume. The reconstruction is based on an applied excitation (e.g., current) to transducers surrounding the area or volume, and a measured response (e.g., voltage) acquired at a surface of the area or volume. Visual distributions of the estimates can then be formed.

In conventional soft-field tomography, a single, single-phase excitation (e.g., electrical voltage or current) may be used to determine soft-field tomography parameters. Alternatively, multiple and/or simultaneous excitations may be used. However, these conventional soft-field excitation techniques suffer from a poor signal to noise ratio (SNR) for the detection and quantification of phases, for example, determining the phase fraction of different materials, such as a solid, liquid, gas or combinations thereof. Thus, the resolution for measurements using these systems is low and may be unacceptably low for certain applications. Accordingly, EIS reconstructions of conductivity distributions using these known excitation methods may not provide sufficient resolution.

### BRIEF DESCRIPTION

In accordance with an embodiment, a method for acquiring soft-field data is provided. The method includes applying a plurality of phase modulated (or phase and amplitude modulated) excitations to a plurality of transducers of a data acquisition system positioned proximate a surface of an object and measuring a response to the applied phase modulated excitations at the plurality of transducers. The method also includes determining a property of the object based on the measured responses.

In accordance with another embodiment, a soft-field data acquisition system is provided that includes a plurality of transducers configured for positioning proximate a surface of an object and one or more excitation drivers coupled to the plurality of transducers and configured to generate excitation signals for the plurality of transducers. The excitation signals are phase modulated excitations (or phase and amplitude modulated excitations). The soft-field tomography system also includes one or more response detectors coupled to the plurality of transducers and configured to measure a response of the object at the plurality of transducers to the excitation applied by the plurality of transducers based on the excitation signals. The soft-field tomography system further includes a soft-field reconstruction module configured to reconstruct a property distribution based on the excitation signals and the measured response.

In accordance with yet another embodiment, a computer readable storage medium for acquiring soft-field data and reconstructing a property distribution of an object using a processor is provided. The computer readable storage medium includes instructions to command the processor to apply a plurality of phase modulated excitations (or phase and amplitude modulated excitations) to a plurality of transducers of a soft-field tomography system positioned proximate a surface of an object and measure a response to the applied phase modulated excitations (or phase and amplitude modulated excitations) at the plurality of transducers. The computer readable storage medium also includes instructions to command the processor to determine a spatial property distribution within the object based on the measured response.

### BRIEF DESCRIPTION OF THE DRAWINGS

The presently disclosed subject matter will be better understood from reading the following description of non-limiting embodiments, with reference to the attached drawings, wherein below:
Figure 1 is a simplified block diagram illustrating a soft-field tomography system formed in accordance with various embodiments.
Figure 2 is a simplified diagram illustrating excitations using one transducer configuration in accordance with various embodiments.
Figure 3 is a graph illustrating phase or phase and amplitude modulated excitations signals generated in accordance with one embodiment.
Figure 4 is a block diagram illustrating soft-field tomography information flow in accordance with various embodiments.
Figure 5 is a simplified diagram illustrating reconstruction of a property distribution.
Figure 6 is a simplified block diagram illustrating excitation signal generation and data acquisition in accordance with an embodiment.
Figure 7 is a flowchart of a method to generate excitations for transducers of a soft-field tomography system in accordance with various embodiments.

### DETAILED DESCRIPTION

The foregoing summary, as well as the following detailed description of certain embodiments, will be better understood when read in conjunction with the appended drawings. To the extent that the figures illustrate diagrams of the functional blocks of various embodiments, the functional blocks are not necessarily indicative of the division between hardware circuitry. Thus, for example, one or more of the functional blocks (e.g., processors, controllers, circuits or memories) may be implemented in a single piece of hardware or multiple pieces of hardware. It should be understood that the various embodiments are not limited to the arrangements, component/element interconnections and instrumentality shown in the drawings.

As used herein, a module or step recited in the singular and proceeded with the word "a" or "an" should be understood as not excluding plural of said elements or steps, unless such exclusion is explicitly stated. Furthermore, references to "one embodiment" are not intended to be interpreted as excluding the existence of additional embodiments that also incorporate the recited features. Moreover, unless explicitly stated to the contrary, embodiments "comprising" or "having" a module or a plurality of modules having a particular property may include additional such modules not having that property.

Various embodiments provide a system and method for generating soft-field tomography excitations and reconstructing property distributions within an object. For example, excitations are generated for a plurality of transducers arranged proximate to or along a boundary or surface of object in soft-field tomography systems, such as Electrical Impedance Spectroscopy (EIS) or Electrical Impedance Tomography (EIT) systems. However, the various embodiments may apply to other soft-field tomography systems, such as Diffuse Optical Tomography (DOT), Near InfraRed Spectroscopy (NIRS), elastography, thermography or microwave tomography, and related modalities. A technical effect of at least one embodiment is increased signal to noise ratio (SNR) and improved spatial precision for soft-field tomography detection, which can be used to quantify the distribution of different materials, for example, in real time.

It should be noted that as used herein, "soft-field tomography" refers generally to any tomographic or multidimensional extension of a tomographic method that is not "hard-field tomography". Additionally, as used herein, soft-field tomography excitations refer in various embodiments to applying a set of orthonormal basis functions to interrogate a volume or area of interest. Thus, in various embodiments, a set of excitations are applied that match or approximate the modes of the system under test or examination.

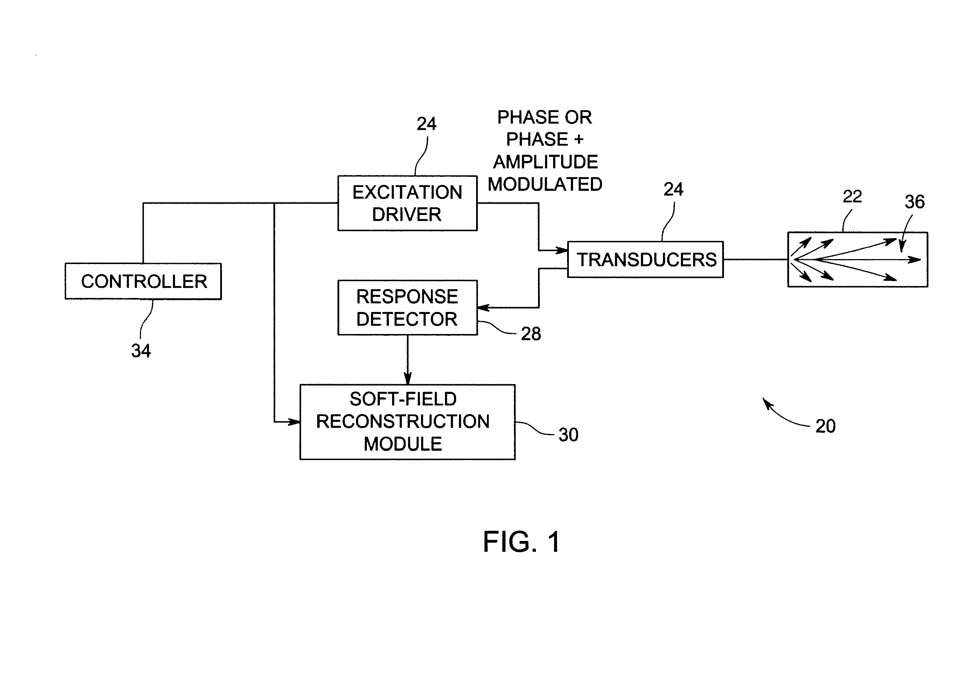

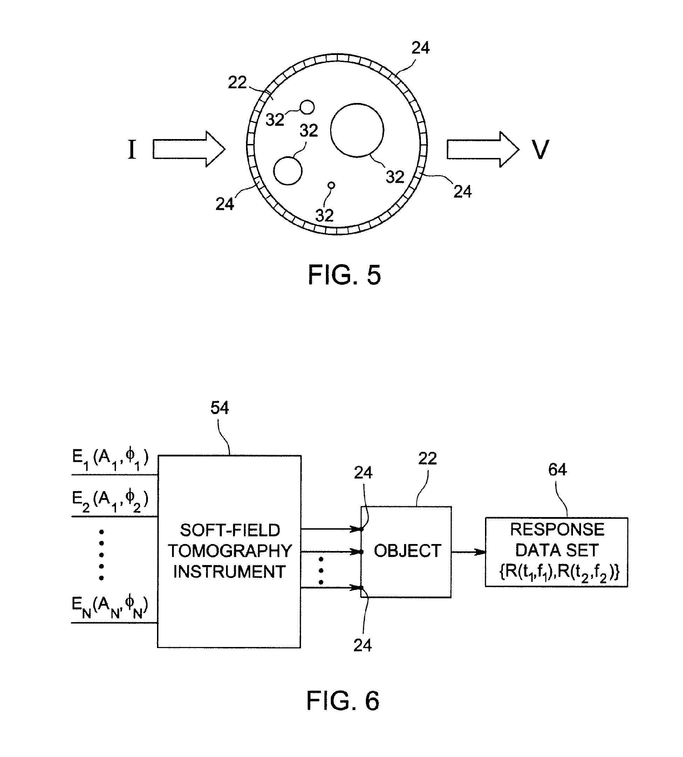

One embodiment of a soft-field tomography system 20 is illustrated in Figure 1. For example, the soft-field tomography system 20 may be an Electrical Impedance Spectroscopy (EIS) system, also referred to as Electrical Impedance Tomography (EIT) system used to determine the electrical properties of materials within an object 22 as illustrated in Figure 5. For example, the spatial distribution of electrical conductivity (σ) and/or permittivity (ε) may be determined inside the object 22 or other area or volume. Thus, internal properties of the object 22 (e.g., a patient) may be determined. In various embodiments, the soft-field tomography system 20 may be, for example, a data acquisition system, such as an EIS or EIT data acquisition system.

In the illustrated embodiment, the system 20 includes a plurality of transducers 24 (e.g., electrodes) that are positioned at or proximate a surface of the object 22, which in a healthcare application (e.g., patient monitoring or tissue characterization) may include attaching the plurality of the transducers 24 to the skin of a patient or subject. For example, the transducers 24 may be positioned on the surface of the object 22 (e.g. electrodes, thermal sources, ultrasound transducers), near the surface of the object 22 (e.g., radiofrequency antenna), or penetrating the surface of the object 22 (e.g., needle electrodes). Thus, the transducers 24 may take different forms, such as surface-contacting electrodes, standoff electrodes, capacitively coupled electrodes, conducting coils, and antennas, among others.

An excitation driver 26 and a response detector 28 are coupled (directly or indirectly through other components) to the transducers 24, which are each connected (directly or indirectly through other components) to a soft-field reconstruction module 30. The soft-field reconstruction module 30 may be any type of processor or computing device that performs soft-field reconstruction based at least in part on received responses from the transducers 24 and that are excited using amplitude (resonant) and phase-modulated excitations as described in more detail herein. The soft-field reconstruction module 30 may be hardware, software or a combination thereof. In one embodiment, the excitation driver 26 and the response detector 28 are physically separate devices. In other embodiments, the excitation driver 26 and the response detector 28 are physically integrated as one element. A controller 34 is also provided and sends instructions to the excitation driver 26 that drives the transducers 24 based on the instructions. It should be noted that an excitation driver 26 may be provided in connection with all of the transducers 24 or a subset of the transducers 24.

It also should be noted that different types of excitations may be used to obtain property distribution data for use in the reconstruction process. For example, electrical, magnetic, optical, thermal or ultrasound excitations, among others, may be used in combination with the various embodiments. In these different embodiments, the transducers 24 may be coupled to the object 22 in different ways and not necessarily in direct contact or only at a surface of the object 22 (e.g., coupled electrically, capacitively, galvanically, etc.).

In one embodiment, the object 22 is a human body region, such as a head, a chest, or a leg, wherein air, blood, muscle, fat, and other tissues have different electrical conductivities. The soft-field tomography system 20 estimates or determines conditions of the internal properties (e.g., material properties) of the human body region, and thus can assist in the diagnoses of diseases, for example, associated with hemorrhage, tumor, and lung function, among others. The object is not limited to humans and animals as non-living objects are also subject to the techniques detailed herein. For example, the excitations of the various embodiments may be applied to an object formed from solids, liquids and/or plasmas (or combinations thereof). In other embodiments, the soft-field tomography system 20 can be used for generating a visual representation of the electrical impedance distribution in a variety of other applications, such as industrial applications, for example, for determining the material properties in a mixed flow including oil and water, or for an underground earth area for soil analysis and roadbed inspection, among others.

It should be noted that any suitable soft field tomography method for generating a distribution of properties of the internal structure of the object 22 may be used, such as with the soft-field reconstruction module 30 defining a geometry of the object 22, and discretizing the geometry into a structure having a plurality of nodes and elements.

In various embodiments, the transducers 24 are formed from any suitable material. For example, the types of transducer 24 used may be based on the particular application, such that a corresponding transducer type (e.g., electrode, coil, etc.) is used to generate the soft-field excitations (e.g., electromagnetic field) and receive responses of the object to the excitations for the particular application. In some embodiments, a conductive material may be used to establish electrical current. For example, the transducers 24 may be formed from one or more metals such as copper, gold, platinum, steel, silver, and alloys thereof. Other exemplary materials for forming the transducers 24 include non-metals that are electrically conductive, such as a silicon based materials used in combination with micro-circuits. In one embodiment where the object 22 is a human body region, the transducers 24 are formed from silver-silver chloride. Additionally, the transducers 24 may be formed in different shapes and/or sizes, for example, as rod-shaped, flat plate-shaped, or needle-shaped structures. It should be noted that in some embodiments, the transducers 24 are insulated from one another. In other embodiments, the transducers 24 can be positioned in direct ohmic contact with the object 22 or be capacitively coupled to the object 22.

In operation, the transducers 24 or a subset of the transducers 24 may be used to transmit signals (e.g., deliver or modulate signals), for example, deliver electrical current continuously such that excitations may be applied across a temporal or varying frequency range (e.g., 1 kHz to 1 MHz), such as over multiple carrier frequencies (e.g., temporal frequencies), and which is also amplitude modulated, to the object 22 to generate an electromagnetic (EM) field within the object 22. In an EIS or EIT application, the resulting surface potentials, namely the voltages on the transducers 24 are measured to determine an electrical conductivity or permittivity distribution using one or more suitable reconstruction methods. For example, a visual distribution may be reconstructed based on the geometry of the transducers 24, the applied currents and the measured voltages.

Thus, in various embodiments, the excitation driver 26 applies an excitation to each of the transducers 24 and the response detector 28 measures a response of the object 22 at each of the transducers 24 (which may be multiplexed by a multiplexer) in response to the excitation applied on the transducers 24. It should be noted that any type of excitation may be provided, for example, electrical current, electrical voltage, a magnetic field, a radio-frequency wave, a thermal field, an optical signal, a mechanical deformation and an ultrasound signal, among others. For example, the excitation driver 26 may apply (i) phase varying/modulated or (ii) phase and amplitude varying/modulated electrical voltage or current signals to the object 22 that has different electrical and magnetic material properties, such as conductivity, permittivity and/or permeability.

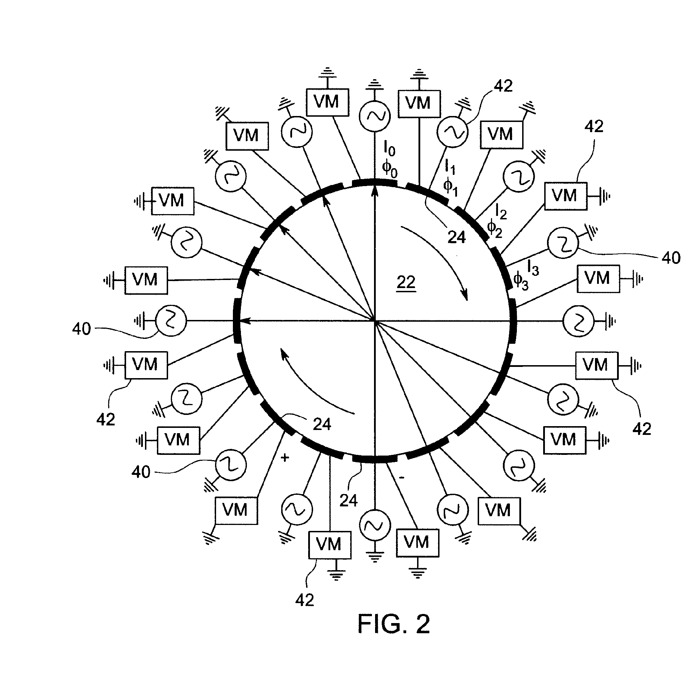

In various embodiments, the combined amplitude and phase modulated signals applied by the excitation driver 26 to the transducers 24 generate a rotating field 36 (that rotates in time and space), illustrated for simplicity by the arrows within the object 22 shown in Figure 1. For example, the field in various embodiments may rotate (as a result of phase modulation) and optionally also increase or decrease over time (as a result of amplitude modulation). The resonant rotating field 36 generated by the phase modulated excitations applied at the transducers 24 is illustrated more specifically in Figure 2. The arrows represent the rotating EM field generated within the object 22. In particular, a different excitation is applied to a plurality of the transducers 24, which may be a subset or all of the transducers 24. For example, the excitation driver 26 (shown in Figure 1) applies an excitation pattern on the geometry by applying a phase varying excitation (or phase and amplitude varying excitation) 40 on each of the transducers 24. Thus, each transducer 24 has applied thereto an excitation having a varying phase or amplitude (e.g., +/- 1 milliamp) with a different phase or associated angle (e.g., a one degree difference) that results in the rotating EM field 36. It should be noted that the excitation pattern and measured response are simplified for illustration and the excitation and conductivity distribution may be more complex.

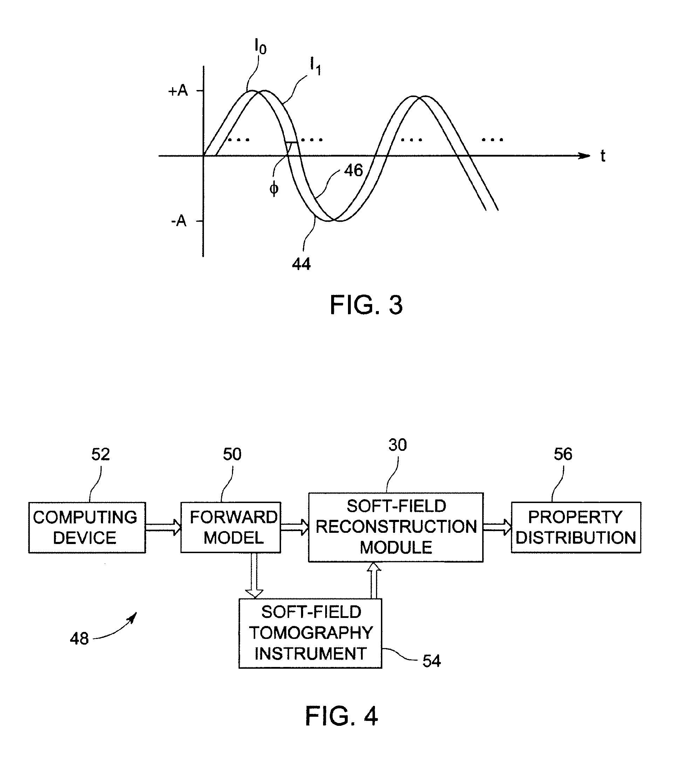

The illustrated excitation is an amplitude and phase varying electrical current that may be defined as follows: I₁ = A₁sin Ω(t) + Φ₁, wherein I₁ is the excitation (e.g., electrical, microwave, optical, magnetic, thermal, or radio-frequency, signals among others) on the 1^{th} transducer 24, A₁ is the magnitude or amplitude of the excitation and Φ is the phase on the 1^{th} transducer 24, and which may be different for each of the transducers 24. It should be noted that the modulated current I₁ applied at each of the transducers 24 may have the same amplitude or different amplitudes. Figure 3 illustrates two excitations, namely I₁ and I₂ that may be applied to two different transducers 24. As can be seen by the waveforms 44 and 46, illustrated as, but not limited to generally sinusoidal signals, the amplitudes of the signals may vary by the same amount or different amounts, and the phase of each is different by Φ, which may be the same or different. It should be noted that the shape of the signals may be varied as desired or needed, for example, to have larger/smaller slopes, wider/narrower peaks, etc. Thus, an increasing and decreasing field strength are applied, while the EM field is rotating. It should be noted that the excitations may be performed using different types of signals, such as multi-sine or other composite waveforms.

Referring again to Figure 2, the response detector 28 is illustrated as having a plurality of voltage measuring devices, such as voltmeters 42, for measuring a voltage at the surface of the object 22 at the transducers 24. However, different measurement devices may be used, for example, based on the type of application or object 22.

Using various embodiments, soft-field reconstruction is provided that may be used to determine the material properties of the object 22 using responses from amplitude and phase varying/modulated excitations. Using responses at different times and/or frequencies that correspond to specific material properties, the distribution of the different materials within the object 22 may be reconstructed.

In operation, the soft-field reconstruction module 30, thus, computes a response of the object 22 to the applied excitation. For example, an EIS information flow 48 is illustrated in Figure 4. In particular, a forward model 50 is used based on excitations from a computing device 52, to predict responses (predicted data), which are provided to the soft-field reconstruction module 30. In one embodiment, an inverse problem relating the measured responses (e.g., measured signals), and the applied excitations, and the electrical conductivity distribution inside of the object 22 being tested or interrogated by the soft-field tomography system 20 is solved by the reconstruction module 30.

The excitations are applied to the object 22 (shown in Figures 1 and 2) by the soft-field tomography instrument 54, which may include the transducers 24 and other excitation and measurement components, and thereafter measured voltages (measured data) are communicated to the soft-field reconstruction module 30. The soft-field reconstruction module 30 then performs reconstruction using any suitable reconstruction method to generate an estimate of the property distribution 56, for example, the impedance distribution, to identify regions of interest 32 (shown in Figure 5) within the object 22. It should be noted that the various components may be physically separate components or elements or may be combined. For example, the soft-field reconstruction module 30 may form part of the soft-field tomography system 20 (as illustrated in Figure 1). In an EIS or EIT application, and as illustrated in Figure 5, a soft-field reconstruction is performed to identify regions of interest 32 within the object 22. As shown, the response detector 28 (shown in Figure 1) measures a response on the transducers 24 in response to the excitation applied by the excitation driver 26 (shown in Figure 1) to the transducers 24.

In one embodiment, excitations may be generated as illustrated in Figure 6. In particular, the soft-field tomography instrument 54 generates excitations at the transducers 24, which may be excitation currents that are phase modulated or amplitude and phase modulated. For example, a phase-modulated alternating excitation may be applied to the plurality of the transducers 24 to generate a field within the object 22 (shown in Figures 1 and 2) that rotates spatially and temporally. In one embodiment, the excitation applied at each of the transducers 24 has the same varying amplitude, but has a different phase. Additionally, in this embodiment, multiple excitations having the same or different frequencies may be applied over time to each of the transducers 24.

In particular, at a first excitation time T₁, an n-phase modulated excitation (which optionally may be amplitude modulated) is applied to a set of N transducers 24, where N is greater than one. Thereafter, at one or more excitation times T₂...T_{N}, an n-phase modulated (or phase and amplitude modulated) excitation is applied to a set of N transducers 24 (e.g., a different set of transducers 24) until n-phase modulated (or phase and amplitude modulated) excitations are applied to all of the transducers 24. Thus, in various embodiments, a plurality of excitation patterns are applied with all of a subset of the transducers 24 excited simultaneously. Accordingly, an excitation pattern is applied to all of a subset of the transducers 24 that is phase modulated or phase and amplitude modulated. In various embodiments, a spatial frequency is implemented wherein the modulation is provided by transducer location. For example, the amplitude of the excitations may be varied based on a trigonometric function that can generate cosineθ or sineθ components. However, the carrier frequency may be the same (e.g., 1 kHz).

Thus, as illustrated in Figure 6, after a first excitation sequence, which may occur over multiple excitation times, an n-phase modulated (or phase and amplitude modulated) excitation is applied to all or a subset of the transducers 24. In particular, excitation E₁(A₁, Φ₁) is applied to transducer 1, excitation E₁(A₂, Φ₂) is applied to transducer 2.... excitation E_{N}(A_{N}, Φ_{N}) is applied to transducer N, where the phase (or angle) Φ is modulated and the amplitude A also may be modulated. After an excitation sequence has been performed, each of the transducers 24 has a phase modulated excitation (or optionally a phase and amplitude modulated excitation) applied thereto. Thus, in one example, the excitation applied to each of the transducers 24 is a signal having the same varying amplitude A, namely that the magnitude of the amplitude varies the same, but having a different phase Φ. However, in other embodiments the amplitude also varies.

A response is measured at all of the transducers 24 after application of each of the excitations, namely after each of the excitations during an excitation sequence, which may be applied to different subsets of the transducers 24. For example, if all of the transducers 24 are excited at two different excitation times that define a single excitation sequence, first and second responses are measured after the application of the first and second excitation times (t₁ and t₂) to generate a response data set 60. The first and second excitations, thus, are phase modulated (or optionally phase and amplitude modulated) excitations that generate an EM field that rotates in time and space. The multiple responses may be analyzed or combined to reconstruct the distribution of different materials. For example, the responses in the response data set 60 may be combined using an suitable combination process, such as an additive or scaling process, among others.

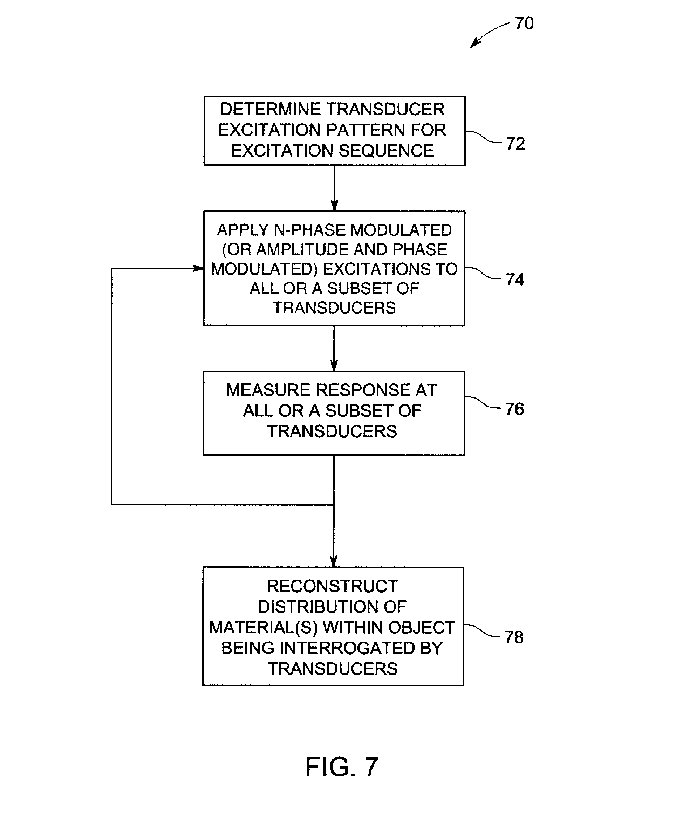

In various embodiments, a method 70 as illustrated in Figure 7 is provided to generate excitations for transducers of a soft-field tomography system, such as the transducers 24. The method 70 may be implemented in connection with a soft-field tomography system that includes several transducers (e.g., electrodes). The method 70 includes determining at 72 a transducer excitation pattern for an excitation sequence to be used to interrogate an object using the transducers. For example, a determination may be made based on the type of material properties to be determined, the type of object to be examined/interrogated, etc., the particular transducers or sets of transducers to be excited and the pattern or order or excitation. In one embodiment, the selection of "N" transducers or sets of transducers to excite is based on a logic implementation, which may be based on the intended application and system requirements, such that resolution, data acquisition, noise performance (e.g., signal-to-noise ratio), detection capability, complexity or acquisition speed optimized or improved, while reducing or minimizing the required analysis in determining the materials/compositions. In another embodiment, the selection of "N" transducers or sets of transducers to excite is based on an optimization using signal-to-noise ratio, instrumentation complexity and acquisition speed, which may be application dependent.

Thereafter, n-phase modulated (or phase and amplitude modulated) excitations are applied to all or a subset of transducers at 74. In some embodiments, the excitations may be applied to all of the transducers. As one example, at a first excitation time of an excitation sequence, a set of "N" electrodes is supplied with n-phase modulated (or phase and amplitude modulated) excitations, for example, electrical signals (e.g., voltage or current) distributed in space (symmetrically or asymmetrically in space and time). As the excitations are applied, the responses (e.g., resulting voltage or current signals) through or at all of a subset of the "N" transducers are measured at 76.

It should be noted that steps 74 and 76 are optionally repeated until all derived excitation patterns are applied to all or a subset of the transducers and responses measured. Thus, this process is continued until excitations have been applied to all of the transducers at least once.

Once all of the transducers have been provided with an excitation signal, thereby completing the excitation sequence, a distribution of the material(s) within the object being interrogated by the transducers is reconstructed at 78. For example, any suitable EIS/EIT reconstruction technique may be used.

As an example of one application, and in one embodiment, phase or phase and amplitude modulated electrical impedance tomography may be performed using the various embodiments. For example, the various embodiments may be used for various medical applications, such as monitoring of lung function, detection of cancer in the skin and breast and location of epileptic foci. As another example, and in another embodiment, phase or phase and amplitude modulated electrical impedance tomography may be performed using the various embodiments for applications where the presence of a foreign material is to be detected real-time across the cross-section of the body, such as flow sensing applications, mold filling visualization in casting, chemical and other process engineering applications.

As still another example, and in another embodiment, phase or phase and amplitude modulated electrical impedance tomography may be performed using the various embodiments in connection with inspection applications where the presence of a foreign material or loss of material is to be detected across the cross-section of piece under inspection. Some examples of applications include the inspection of composites, the inspection of metals (e.g., welds, etc.), polymers, material loss along a thickness of a pipe, etc.

Thus, in various embodiments, phase or phase and amplitude modulated electrical impedance tomography may be performed for measuring and determining the permittivity or conductivity variations across a cross-section of a specimen under test that can be applied to detect and quantify the distribution of the different materials real-time.

The various embodiments and/or components, for example, the modules, elements, or components and controllers therein, also may be implemented as part of one or more computers or processors. The computer or processor may include a computing device, an input device, a display unit and an interface, for example, for accessing the Internet. The computer or processor may include a microprocessor. The microprocessor may be connected to a communication bus. The computer or processor may also include a memory. The memory may include Random Access Memory (RAM) and Read Only Memory (ROM). The computer or processor further may include a storage device, which may be a hard disk drive or a removable storage drive such as an optical disk drive, solid state disk drive (e.g., flash RAM), and the like. The storage device may also be other similar means for loading computer programs or other instructions into the computer or processor.

As used herein, the term "computer" or "module" may include any processor-based or microprocessor-based system including systems using microcontrollers, reduced instruction set computers (RISC), application specific integrated circuits (ASICs), field-programmable gate arrays (FPGAs), graphical processing units (GPUs), logic circuits, and any other circuit or processor capable of executing the functions described herein. The above examples are exemplary only, and are thus not intended to limit in any way the definition and/or meaning of the term "computer".

The computer or processor executes a set of instructions that are stored in one or more storage elements, in order to process input data. The storage elements may also store data or other information as desired or needed. The storage element may be in the form of an information source or a physical memory element within a processing machine.

The set of instructions may include various commands that instruct the computer or processor as a processing machine to perform specific operations such as the methods and processes of the various embodiments of the invention. The set of instructions may be in the form of a software program, which may form part of a tangible non-transitory computer readable medium or media. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to operator commands, or in response to results of previous processing, or in response to a request made by another processing machine.

As used herein, the terms "software", "firmware" and "algorithm" are interchangeable, and include any computer program stored in memory for execution by a computer, including RAM memory, ROM memory, EPROM memory, EEPROM memory, and non-volatile RAM (NVRAM) memory. The above memory types are exemplary only, and are thus not limiting as to the types of memory usable for storage of a computer program.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the various embodiments of the invention without departing from their scope. While the dimensions and types of materials described herein are intended to define the parameters of the various embodiments of the invention, the embodiments are by no means limiting and are exemplary embodiments. Many other embodiments will be apparent to those of skill in the art upon reviewing the above description. The scope of the various embodiments of the invention should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects. Further, the limitations of the following claims are not written in means-plus-function format and are not intended to be interpreted based on 35 U.S.C. § 112, sixth paragraph, unless and until such claim limitations expressly use the phrase "means for" followed by a statement of function void of further structure.

This written description uses examples to disclose the various embodiments of the invention, including the best mode, and also to enable any person skilled in the art to practice the various embodiments of the invention, including making and using any devices or systems and performing any incorporated methods. The patentable scope of the various embodiments of the invention is defined by the claims, and may include other examples that occur to those skilled in the art. Such other examples are intended to be within the scope of the claims if the examples have structural elements that do not differ from the literal language of the claims, or if the examples include equivalent structural elements with insubstantial differences from the literal languages of the claims.

Various aspects and embodiments of the present invention are defined by the following numbered clauses:
1. A method for acquiring soft-field data, the method comprising:
   applying a plurality of phase modulated excitations to a plurality of transducers of a soft-field data acquisition system positioned proximate a surface of an object;
   measuring a response to the applied phase modulated excitations at the plurality of transducers; and
   determining a property of the object based on the measured responses.
2. The method of clause 1, wherein the plurality of excitations are n-phase modulated excitations and further comprising applying the n-phase modulated excitations to at least a subset of the plurality of transducers.
3. The method of clause 1 or clause 2, further comprising applying the n-phase modulated excitations to at least another subset of the plurality of transducers until excitations have been applied to all of the plurality of transducers.
4. The method of any preceding clause, further comprising applying the n-phase modulated excitations during a excitation sequence, wherein the excitation sequence is based on an application for the soft-field tomography system, which is optimized using at least one of signal-to-noise ratio, resolution, detection capability, instrumentation complexity or acquisition speed.
5. The method of any preceding clause, further comprising applying n-phase modulated excitations over multiple excitation sequences over time at different temporal frequencies.
6. The method of any preceding clause, further comprising applying n-phase modulated excitations to subsets of the plurality of transducers using one or more of a sinusoidal waveform, a multisine waveform or a composite waveform.
7. The method of any preceding clause, further comprising amplitude modulating the excitations applied to the plurality of transducers.
8. The method of any preceding clause, wherein a phase of the phase modulated excitation applied to the plurality of transducers is different for each of the plurality of transducers.
9. The method of any preceding clause, further comprising performing a temporal frequency scan of the object to determine one or more properties at the different frequencies to reconstruct the spatial distribution of a plurality of materials within the object.
10. The method of any preceding clause, wherein the phase modulated excitations comprise one or more of electrical signals, optical signals, magnetic signals, thermal signals, radio-frequency signals or microwave signals distributed in space symmetrically or asymmetrically in space and time.
11. The method of any preceding clause, wherein the spatial property distribution is a distribution as determined in one of Electrical Impedance Spectroscopy (EIS), Electrical Impedance Tomography (EIT), Diffuse Optical Tomography (DOT), Near InfraRed Spectroscopy (NIRS), thermography, elastography or microwave tomography.
12. The method of any preceding clause, wherein the spatial property distribution comprises a distribution of one or more of electric conductivity, electric permittivity, magnetic permeability, optical absorbance, optical scattering, optical reflectivity, elasticity, or thermal conductivity.
13. The method of any preceding clause, wherein determining the property of the object comprises determining a spatially distributed property of the object.
14. A soft-field data acquisition system comprising:
   a plurality of transducers configured for positioning proximate a surface of an object;
   one or more excitation drivers coupled to the plurality of transducers and configured to generate excitation signals for the plurality of transducers, wherein the excitation signals comprise phase modulated excitations;
   one or more response detectors coupled to the plurality of transducers and configured to measure a response of the object at the plurality of transducers to the excitation applied by the plurality of transducers based on the excitation signals; and
   a soft-field reconstruction module configured to reconstruct a property distribution based on the excitation signals and the measured response.
15. The soft-field data acquisition system of any preceding clause, wherein the one or more excitation drivers are configured to generate n-phase modulated excitations and apply the n-phase modulated excitations to at least a subset of the plurality of transducers.
16. The soft-field data acquisition system of any preceding clause, wherein the one or more excitation drivers are configured to generate the n-phase modulated excitations and apply the n-phase modulated excitations to at least another subset of the plurality of transducers until excitations have been applied to all of the plurality of transducers.
17. The soft-field data acquisition system of any preceding clause, wherein the one or more excitation drivers are configured to apply the n-phase modulated excitations during an excitation sequence, wherein the excitation sequence is based on an application that is optimized using at least one of signal-to-noise ratio, resolution, detection capability, instrumentation complexity or acquisition speed.
18. The soft-field data acquisition system of any preceding clause, wherein the one or more excitation drivers are configured to apply n-phase modulated excitations over multiple excitation sequences over time at different temporal frequencies.
19. The soft-field data acquisition system of any preceding clause, wherein the one or more excitation drivers are configured to apply n-phase modulated excitations to subsets of the plurality of transducers using one or more of a sinusoidal waveform, a multisine waveform or a composite waveform.
20. The soft-field data acquisition system of any preceding clause, wherein the property distribution is a distribution as determined in one or more of Electrical Impedance Spectroscopy (EIS), Electrical Impedance Tomography (EIT), Diffuse Optical Tomography (DOT), Near InfraRed Spectroscopy (NIRS), thermography, elastography or microwave tomography.
21. The soft-field data acquisition system of any preceding clause, wherein the property distribution comprises a distribution of one or more of electric conductivity, electric permittivity, magnetic permeability, optical absorbance, optical scattering, optical reflectivity, elasticity, or thermal conductivity.
22. The soft-field data acquisition system of any preceding clause, wherein the one or more excitation drivers are configured to generate n-phase and amplitude modulated excitations and apply the n-phase and amplitude modulated excitations to at least a subset of the plurality of transducers.
23. A computer readable storage medium for acquiring soft-field data and reconstructing a property distribution of an object using a processor, the computer readable storage medium including instructions to command the processor to:
   apply a plurality of phase modulated excitations to a plurality of transducers of a soft-field tomography system positioned proximate a surface of an object;
   measure a response to the applied phase modulated excitations at the plurality of transducers; and
   determine a property of the object based on the measured response.
24. The computer readable storage medium of any preceding clause, wherein the plurality of excitations are n-phase modulated excitations, and the instructions command the processor to further apply the n-phase synchronized excitations to at least a subset of the plurality of transducers.
25. The computer readable storage medium of any preceding clause, wherein the instructions command the processor to further apply the n-phase modulated excitations to at least another subset of the plurality of transducers until excitations have been applied to all of the plurality of transducers.
26. The computer readable storage medium of any preceding clause, wherein the instructions command the processor to further apply the n-phase modulated excitations during an excitation sequence, wherein the excitation sequence is based on an application for the soft-field tomography system, which is optimized using at least one signal-to-noise ratio, resolution, detection capability, instrumentation complexity or acquisition speed.
27. The computer readable storage medium of any preceding clause, wherein the instructions command the processor to further apply n-phase modulated excitations over multiple excitation sequences over time at different temporal frequencies.
28. The computer readable storage medium of any preceding clause, wherein the instructions command the processor to further apply n-phase modulated excitations to subsets of the plurality of transducers using one or more of a sinusoidal waveform, a multisine waveform or a composite waveform.
29. The computer readable storage medium of any preceding clause, wherein the instructions command the processor to further generate n-phase and amplitude modulated excitations and apply the n-phase and amplitude modulated excitations to at least a subset of the plurality of transducers.
30. The computer readable storage medium of any preceding clause, wherein the instructions command the processor to further determine a spatially distributed property of the object based on the measured response.

## Claims

1. A method for acquiring soft-field data, the method comprising:
applying a plurality of phase modulated excitations to a plurality of transducers of a soft-field data acquisition system positioned proximate a surface of an object;
measuring a response to the applied phase modulated excitations at the plurality of transducers; and
determining a property of the object based on the measured responses.

2. The method of claim 1, wherein the plurality of excitations are n-phase modulated excitations and further comprising applying the n-phase modulated excitations to at least a subset of the plurality of transducers.

3. The method of claim 1 or claim 2, further comprising applying the n-phase modulated excitations to at least another subset of the plurality of transducers until excitations have been applied to all of the plurality of transducers.

4. The method of any preceding claim, further comprising applying the n-phase modulated excitations during a excitation sequence, wherein the excitation sequence is based on an application for the soft-field tomography system, which is optimized using at least one of signal-to-noise ratio, resolution, detection capability, instrumentation complexity or acquisition speed.

5. The method of any preceding claim, further comprising applying n-phase modulated excitations over multiple excitation sequences over time at different temporal frequencies.

6. The method of any preceding claim, further comprising applying n-phase modulated excitations to subsets of the plurality of transducers using one or more of a sinusoidal waveform, a multisine waveform or a composite waveform.

7. The method of any preceding claim, further comprising amplitude modulating the excitations applied to the plurality of transducers.

8. The method of any preceding claim, wherein a phase of the phase modulated excitation applied to the plurality of transducers is different for each of the plurality of transducers.

9. The method of any preceding claim, further comprising performing a temporal frequency scan of the object to determine one or more properties at the different frequencies to reconstruct the spatial distribution of a plurality of materials within the object.

10. The method of any preceding claim, wherein the phase modulated excitations comprise one or more of electrical signals, optical signals, magnetic signals, thermal signals, radio-frequency signals or microwave signals distributed in space symmetrically or asymmetrically in space and time.

11. The method of any preceding claim, wherein the spatial property distribution is a distribution as determined in one of Electrical Impedance Spectroscopy (EIS), Electrical Impedance Tomography (EIT), Diffuse Optical Tomography (DOT), Near InfraRed Spectroscopy (NIRS), thermography, elastography or microwave tomography.

12. The method of any preceding claim, wherein the spatial property distribution comprises a distribution of one or more of electric conductivity, electric permittivity, magnetic permeability, optical absorbance, optical scattering, optical reflectivity, elasticity, or thermal conductivity.

13. The method of any preceding claim, wherein determining the property of the object comprises determining a spatially distributed property of the object.

14. A soft-field data acquisition system comprising:
a plurality of transducers configured for positioning proximate a surface of an object;
one or more excitation drivers coupled to the plurality of transducers and configured to generate excitation signals for the plurality of transducers, wherein the excitation signals comprise phase modulated excitations;
one or more response detectors coupled to the plurality of transducers and configured to measure a response of the object at the plurality of transducers to the excitation applied by the plurality of transducers based on the excitation signals; and
a soft-field reconstruction module configured to reconstruct a property distribution based on the excitation signals and the measured response.

15. A computer readable storage medium for acquiring soft-field data and reconstructing a property distribution of an object using a processor, the computer readable storage medium including instructions to command the processor to:
apply a plurality of phase modulated excitations to a plurality of transducers of a soft-field tomography system positioned proximate a surface of an object;
measure a response to the applied phase modulated excitations at the plurality of transducers; and
determine a property of the object based on the measured response.
